(19) Europäisches Patentamt

European Patent Office

Office européen des brevets

(11) **EP 0 182 520 B2**

(12) **NEW EUROPEAN PATENT SPECIFICATION**

(45) Date of publication and mention
of the opposition decision:
**28.08.2002  Bulletin 2002/35**

(45) Mention of the grant of the patent:
**25.03.1992  Bulletin 1992/13**

(21) Application number: **85307771.7**

(22) Date of filing: **28.10.1985**

(51) Int Cl.⁷: **A61N 1/30**

(54) **Apparatus for iontophoretic drug delivery**

Gerät zur iontophoretischen Verabreichung von Medikamenten

Appareil d'application iontophorétique de médicaments

(84) Designated Contracting States:
**DE FR GB NL**

(30) Priority: **29.10.1984  US 665698**
**29.10.1984  US 665699**

(43) Date of publication of application:
**28.05.1986  Bulletin 1986/22**

(73) Proprietor: **ALZA CORPORATION**
**Palo Alto California 94303-0802 (US)**

(72) Inventors:
• **Unterecker, Darrel F.**
  **Cedar Minnesota 55011 (US)**
• **Phipps, Joseph B.**
  **Plymouth Minnesota 55442 (US)**

(74) Representative: **Cockbain, Julian, Dr. et al**
**Frank B. Dehn & Co.,**
**European Patent Attorneys,**
**179 Queen Victoria Street**
**London EC4V 4EL (GB)**

(56) References cited:
**EP-A- 0 058 920**        **EP-A- 0 097 436**
**US-A- 4 383 529**        **US-A- 4 474 570**

• **Samarin et al, Physicochemical Study of Iontophoresis, Vosprosy Kurotologii, Fizioterapi i Lechebnoi Fizicheskoi Kul tury 1957, no 4, pages 3 to 7**
• **Chapter 12, "Iontophoresis", P.A.Rebinder, ed, pages 310 to 327, Moscow,USSR, Acadamy of Science, 1956**
• **J. Allergy Clin. Immunol, vol.52, no 6, 1973, pages 328 to 333, R.H. Shereff et al: "Effect of beta adrenergic stimulation and blockade on immediate hypersensitivity skin test reactions".**

**EP 0 182 520 B2**

**Description**

This invention relates to improvements in iontophoretic drug delivery, particularly to iontophoretic drug delivery apparatus for transdermal medicament delivery and to improvements therein. More specifically, this invention relates to improved apparatus for active (as opposed to passive) transdermal, ambulatory, drug delivery. Yet more particularly, this invention relates to increased efficiency iontophoresis devices or appliances.

Iontophoresis, according to <u>Dorland's Illustrated Medical Dictionary,</u> is defined to be "the introduction, by means of electric current, of ions of soluble salts into the tissues of the body for therapeutic purposes." Iontophoretic devices have been known since the early 1900's. GB-A-410,009(1934) describes an iontophoretic device which overcame one of the disadvantages of such early devices known to the art at that time, namely the requirement of a special low tension (low voltage) source of current which meant that the patient needed to be immobilized near such a source. The device of that British specification was made by forming from the electrodes and the material containing the medicament or drug to be delivered transdermally, a galvanic cell which itself produced the current necessary for iontophoretically delivering the medicament. This ambulatory device thus permitted iontophoretic drug delivery with substantially less interference with the patient's daily occupation.

More recently, a number of United States patents have issued in the iontophoresis technology, indicating a renewed interest in this mode of drug delivery.. For example, US-A-3,991,755 issued to Jack A. Vernon <u>et al;</u> US-A-4,141,359 issued to Stephen C. Jacobson <u>et al;</u> US-A-4,390,545 issued to Wilson; US-A-4,250,878 issued to Jacobsen disclose examples of iontophoretic devices and some applications thereof. The iontophoresis process has been found to be useful in the transdermal administration or introduction of medicaments or drugs including lidocaine hydrochloride, hydrocortisone, acetic acid, fluoride, penicillin, dexamethasone sodium phosphate and many other drugs. Perhaps the widest use of iontophoresis is that of diagnosing cystic fibrosis by using pilocarpine nitrate iontophoresis. The pilocarpine nitrate stimulates sweat production; the sweat is collected and analyzed for its chloride content to detect the presence of the disease.

In presently known iontophoretic devices, at least two electrodes are generally used. Both these electrodes are disposed so as to be in intimate electrical contact with some portion of the skin of the body. One electrode, called the active electrode, is the electrode from which the ionic substance, medicament, drug precursor or drug (hereinafter "the ionic drug species") is delivered or driven into the body by electrical repulsion. The other electrode, called the indifferent or ground electrode, serves to close the electrical circuit through the body. In conjunction with the patient's skin contacted by the electrodes, the circuit is completed by connection of the electrodes to a source of electrical energy, e.g. a battery or appropriately modified household current. For example, if the ionic substance to be driven into the body is positively charged, then the positive electrode (the anode) will be the active electrode and the negative electrode (the cathode) will serve to complete the circuit. If the ionic substance to be delivered is negatively charged, then the negative electrode will be the active electrode and the positive electrode will be the indifferent electrode.

Furthermore, existing iontophoresis devices generally require a reservoir or source of the ionized or ionizable species (or a precursor of such species) - ie the ionic drug species - which is to be iontophoretically delivered or introduced into the body. Examples of such reservoirs or sources of ionized or ionizable species include a pouch as described in the previously mentioned Jacobsen US-A-4,250,878, or the pre-formed gel body of US-A-4,383,529 (Webster). Such drug reservoirs, when electrically connected to the anode or the cathode of an iontophoresis device to provide a fixed or renewable source of one or more desired species, are generally used with anodes or cathodes which are substantially electrochemically inert. As is more fully discussed below, utilization of such substantially inert electrodes as contemplated in the prior art generally has significant disadvantages.

It is an object of the present invention to enhance the efficiency of the iontophoretic delivery of ionized or ionizable medicaments e.g., drugs, by means of the selection and utilization of a combination of anode or cathode conductive members or electrodes having specified characteristics and of the drug(s) to be delivered. Further subsidiary objects of the invention were to increase the safety and acceptability of the iontophoretic drug delivery process.

Iontophoretic apparatus and assemblies according to the preamble of the independent claims are described in Samarin et al. Physicochemical Study of Iontophoresis, Vosprosy Kurotologii, Fizioterapi i Lechebnoi Fizicheskoi Kul'tury 1957, No. 4, pages 3-7,and in Chapter 12 in <u>Iontophoresis</u>, P.A. Rebinder, ed., pages 310-327, Moscow, USSR; Academy of Science, 1956.

According to one aspect therefore the present invention provides an iontophoretic drug delivery apparatus as defined in claim 1.

According to another aspect of the present invention there is provided an iontophoretic drug delivery electrode assembly as defined in claim 4.

Viewed from another aspect the present invention provides an iontrophoretic drug delivery electrode assembly as defined in claim 12.

In a preferred embodiment of the invention the electrode assembly of the apparatus includes a component which is electrochemically active during iontophoretic drug delivery to produce a first species which, with a second species employed in or generated during the operation of said apparatus, is capable of interacting to produce a product which is not sufficiently influenced by electrical fields to be iontophoretically introduced.

Briefly the present invention permits a method of iontophoretic drug delivery wherein the drug to be iontophoretically delivered, an electrochemically active component of the drug delivery apparatus, or both, are selected so that during operation of the device, the production of unwanted ionic species may be minimized.

In this invention, the drug to be iontophoretically delivered, an electrochemically active component of the apparatus or both are selected to reduce the formation of unwanted water electrolysis products during operation of the device.

As contemplated herein, an electrochemically active component of an iontophoresis device is a part of the device which is oxidized or reduced during iontophoretic drug delivery or which oxidizes or reduces other available species. The electrochemically active component is an electrical current distribution member of the apparatus

The improved electrode of this invention provides an iontophoretic device which exhibits enhanced coulombic efficiency in drug delivery processes.

The electrode assembly conveniently comprises a reservoir containing the medicament to be iontophoretically delivered, the reservoir being in electrical connection with an electrochemically active component, e.g. an active current distribution member, the species produced from the electrochemically active component during operation of the device interacting with the medicament of the reservoir during iontophoretic drug delivery so as to minimize the formation and delivery of undesired species, the electrochemically active component being in further electrical connection with a source of electrical energy. In a preferred embodiment, the electrode assembly includes means for securing the assembly to skin so as to permit iontophoretic drug delivery therefrom.

The apparatus of the present invention enables a method of iontophoretic drug delivery having enhanced coulombic efficiency to be performed, the method comprising: selecting the ionic species (e.g. a drug) to be iontophoretically delivered, incorporating the ionic species into an electrode assembly (e.g. in its medicament reservoir); selecting the composition or construction of either the anode or the cathode of the iontophoresis apparatus to include an electrochemically active component so that the electrochemical reaction at the anode or the cathode produces species which interact with the ionic species so as to reduce the formation of undesired ions; placing the selected anode or cathode in electrical connection with the ionic species (e.g. in connection with the reservoir) and with a source of electrical energy; and transdermally delivering the selected ionic species into the body while minimizing the formation and transdermal delivery of undesired ions.

The genesis of the present invention lay in the realization that known iontophoretic drug delivery processes have an efficiency in the range of about 5% or less and that iontophoretic drug delivery is largely a diffusion dependent process. This means that approximately 95% of the current utilized in known iontophoresis processes is consumed in activities other than delivering the intended drug. For example, much current is wasted by the migration of highly mobile species such as $H^+$, $OH^-$, $Na^+$ and $Cl^-$.

Thus it was realized that iontophoretic drug delivery efficiency would be enhanced if the availability of species which are more mobile than the drug which was to be delivered were minimized. Minimization of the concentration or charge of species which were more easily transported than the intended species (i.e. a drug) is utilized herein to provide enhanced drug delivery. The enhanced drug delivery described herein may permit iontophoresis to become a viable alternative to other drug delivery techniques.

Prior art iontophoretic devices often utilize substantially inert stainless steel, nickel or other nickel and iron alloys as current distribution members or electrodes. During operation of such iontophoretic devices in accordance with prior art iontophoretic drug delivery methods, electrolysis of water occurs. Hydronium ($H^+$) ions are formed at the anode and hydroxide ions ($OH^-$) are produced at the cathode. In addition, gaseous hydrogen and oxygen are evolved at the cathode and anode, respectively. (Use of nickel or nickel-containing alloys, e.g. stainless steel, also occasionally results in the formation of nickel ions at the anode. The nickel ions are then free to migrate into the drug reservoir of the device and from there into the body.)

Iontophoretic devices which employ such essentially inert electrodes have several inherent disadvantages. First, they exhibit reduced coulombic efficiency of drug transfer (i.e. of charged species) due to the formation of highly mobile hydronium and hydroxide ions at the electrodes. In addition, such devices may evidence instability of pH in the anodic and cathodic reservoirs and adjacent the skin due to the formation of hydronium or hydroxide ions and gaseous products (hydrogen and oxygen) at the electrodes. Lastly, as noted above, while prior art iontophoretic electrodes are substantially electrochemically inert, some undesirable oxidation, e.g. of nickel or other alloy metals, does occur.

These disadvantages mean that known iontophoretic devices used according to conventional drug delivery methods have the following drawbacks:

1) Lower coulombic efficiency requires that the battery for a portable or ambulatory device be larger (i.e. of higher capacity). In addition, the voltage required to maintain a certain therapeutic dose rate becomes larger as the coulombic efficiency becomes smaller.

2) The shift in pH in the cathodic and/or anodic reservoirs, which may for example be in gel form, that is caused by the electrolysis of water may cause skin irritation, possible degradation of the physical properties of the gel components of the apparatus or may change the activity of the drug.

3) The formation of hydrogen and oxygen gas at the electrodes can result in a loss of contact between the electrodes and any cathodic or anodic reservoirs leading to reduced apparatus performance.

4) The oxidation of nickel or other metals at the anode may result in the contamination of the anodic reservoir with metal ions which then are free to migrate into the skin and body with possible deleterious effects.

It is to overcome these disadvantages and increase the overall efficiency of the iontophoresis process that the present invention was made.

One method contemplated by the present invention for reducing the formation of undesirable or undesired hydronium ions at an electrode and to reduce contamination of the drug reservoir due to the oxidation of electrode metal is to select intentionally an electrode (e.g. an anode) comprising an electrochemically active or sacrificial component as defined is anyone of the claims which, when oxidized or reduced during operation of the device, produces a species which immediately reacts with ions (e.g. anions) present in the electrode assembly or available to the electrode, e.g. in the drug reservoir (which also may be specifically selected), to form an insoluble salt or neutral chemical compound "Sacrificial" as that term is used herein means that at least a portion of the electrode (whether anode or cathode) is electrochemically oxidized or reduced during transdermal drug delivery. "Electrochemically active", as the term is used herein, means "sacrificial" as defined above, but includes a material that is not itself oxidized or reduced but which participates in oxidation / reduction. The anion or cation present in the drug reservoir can be present as a separately added material or as the counter-ion to an ionic or cationic drug species to be delivered. Thus, it is seen that the design of the sacrificial of the electrochemically active component of the electrode / drug reservoir system can require the selection of a appropriate electrode material, or electrode construction and/or drug or drug salt.

In an analogous fashion, if a neutral species (rather than one which precipitates from the system) is formed from undesired ions there is again a net removal of unwanted species since neutral species also would not reduce the efficiency of delivery of charged drug species by migrating in response to an electrical potential gradient.

Ferro and ferricyanides of many metals (e.g. Ag, Sn, Zn, Ni, Mn) are insoluble and therefore combination of these metals with ion cyanide doped reservoirs may provide suitable sacrificial anode/drug reservoir systems.

One aspect of the invention is to employ $D_xFe(CN)_6$ (x is an integer greater than 0) where it is desired to transport a drug (D+) species through the skin without cationic competition.

In an analogous practice of this invention, negatively charged, anionic drug species may be more efficiently delivered if an electrochemically active component of the cathode is intentionally selected utilizing the precepts of this invention.

An example of a sacrificial cathode electrochemically active material of this invention is chloridized silver. During device operation, the AgCl on the surface of a silver cathode is decomposed to give silver metal and chloride anion ($AgCl + e^- \rightarrow Ag + Cl^-$). The chloride anion is free to migrate, along with any anionic drug, into the body. In this respect, the Ag/AgCl sacrifical cathode is less efficient than the silver/chloride sacrificial in that chloride ion is produced and is delivered to the body. However, the beneficial aspects of the Ag/AgCl cathode include (a) no hydrogen gas formation and (b) no hydroxyl anions produced at the cathode.

One skilled in this art will recognize that a sacrificial cathode of this generic type generally comprises a metallic salt in contact with a metal cathode. Furthermore, device operation must result in the decomposition fo the metallic salt to form a metal in reduced form plus an anion. If both these conditions are met, in this practice of the present invention anionic drugs are delivered with mitigation of pH changes.

The use of a sacrificial cathode as herein described in conjunction with an anionic drug specifically selected so that the counter-ion will react with the anion being produced at the cathode to form an insoluble salt or neutral compound provides an equivalent method for the control of ion transport as outlined for the anode and anodic reservoir.

An example of an anionic drug (D⁻) which could be intentionally selected for use with an Ag/AgCl cathode (these drugs would likely to dispersed or dissolved in the ion drug reservoir) is silver salicylate. In this system, chloride ions formed at the cathode are free to react with silver or copper ions in the drug reservoir to form an insoluble salt thus allowing the negatively charged D⁻ anion to migrate in to the body without anionic competition.

In yet another method contemplated by the present invention to minimize the formation of hydronium and hydroxide ions which consume current and reduce efficiencies, an intercalation-type cathode or anode is employed. Intercalation electrodes have the property of being capable of reducing alkali metal ions, e.g. sodium and potassium. The use of intercalation-type cathode or anode materials is particularly advantageous in that the formation of hydroxyl and hydronium ion is minimized along with a decline in the production of hydrogen or oxygen gas.

It is to be recognized that electrodes comprising intercalation-type materials may be used either on the anode or the cathode of the iontophoresis device and thus can deliver either positively or negatively charged drugs. This can be illustrated for sodium tungstate where the anodic reaction would be:

$$Na_{1+x}WO_3 \rightarrow Na^+ + Na_xWO_3 + e^-$$

For a cathodic reaction,

$$Na^+ + Na_xWO_3 + e^- \rightarrow Na_{1+x}WO_3.$$

In yet another practice of the instant invention in which the overall coulombic efficiency of a given iontophoretic drug delivery device is enhanced, an amalgam electrode is employed optionally in conjunction with the addition of specific metal cations to the drug reservoir, e.g. optionally with selection of a drug having a specific metal cation. In this practice of the invention the amalgam electrode is consumed during electrochemical discharge of the apparatus, and it reacts with a species intentionally made available to the electochemical oxidation /reduction product of the amalgam, e.g. by adding a selected drug to the system such as in the drug reservoir. Thus, as described above, the amalgam material herein described is electrochemically reacted and undesired, highly mobile, charged species are consumed or removed. As with the intercalation-type cathode materials discussed above, the production of hydroxyl and hydronium ions during electrochemical discharge also is reduced. Again pH stability is obtained, thus increasing the overall stability and efficiency of the drug delivery device.

Stated generally therefore, where the ionic drug species to be delivered iontophoretically is a cation then the counteranion is preferably either a metal ion which in the redox conditions of the electrode assembly can plate onto or be absorbed into the cathode or an anion which reacts with cations generated at the cathode to produce a neutral species (eg water, a solid precipitate, or a neutral complex), or an ionic species of relatively low susceptibility to iontophoretic delivery from the electrode assembly. On the other hand, where the drug species is an anion then its countercation is preferably such as to react with anions generated at the anode to produce a neutral species or an ionic species of relatively low susceptibility to iontophoretic delivery from the electrode assembly.

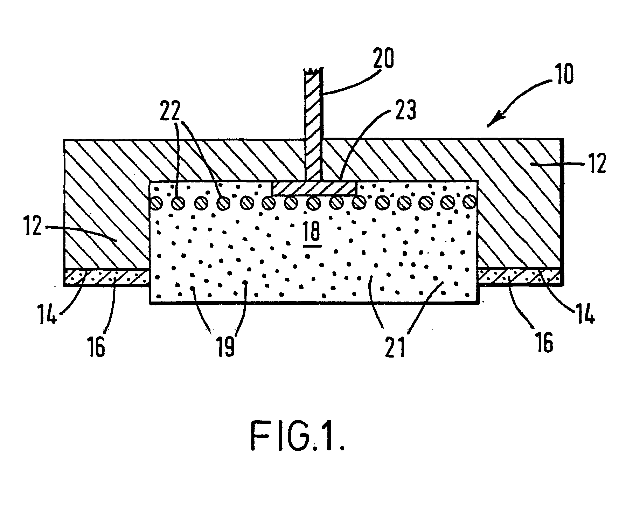

A preferred embodiment of the present invention will now be described by way of example and with reference to the accompanying drawing, Figure 1, which is a cross sectional view of one embodiment of the electrode assembly of the apparatus of the invention.

In Figure 1 there is depicted, schematically, in cross section, a single substantially disc-like electrode assembly 10 which is intended for use in an iontophoretic drug delivery apparatus. It is to be understood that electrode assembly 10 is but one of the two electrodes necessary for successful operation of an iontophoresis device and that the source of electrical energy that may also be required is also not depicted in Figure 1.

Again referring to the figure, electrode assembly 10 comprises a support or housing 12 which is generally "U" shaped in cross section and which is preferably flexible. The support 12 is preferably a flexible backing member, conveniently substantially flat in form. Support 12 may be produced from self-supporting polymeric foam. Perimeter surface 14 of housing 12 would optionally have disposed thereon a skin-compatible, pressure-sensitive, biomedical adhesive 16 which is intended to hold the electrode in place on the patient's skin during iontophoretic drug delivery. The iontophoresis device may be held in place by other means, e.g a strap, in which instance adhesive 16 would not be needed. Thus is it to be understood, as depicted, electrode assembly 10 contemplates drug delivery generally in the direction which appears as downwards in the Figure.

With further reference to the figure, there is shown a drug reservoir 18, which in the illustrated embodiment of the invention is a gel or gel matrix 21 containing the ionic drug species 19 which is to be transdermally introduced across the skin barrier. In a preferred practice of the invention, the self-supporting, skin-compatible gel matrix 21 for the drug would contain sufficient drug 19 so that approximately a one molar solution (applying the definition of a molarity from solvent-solute interactions) would result. Drug concentrations (in the reservoir) in the range of 0.02M to 1.0M or more can conveniently be employed in the practice of this invention. In a preferred practice, the lower reservoir drug concentration ranges (e.g. less than about 0.5 molar) can be used in the efficient devices described herein. It should be noted that any of a number of possible gel matrices may be employed, those being described in the previously mentioned Webster patent comprising a particularly preferred practice of this invention. Agar or polyvinylpyrrolidone gels also are advantageously employed herein.

Also in the figure, there is depicted an exterior connector 20 which in this embodiment is a wire. Exterior connector 20 is in further electrical contact with a current distribution member comprising a tab or plate 23 in electrical contact with an optional screen 22. In this embodiment, the current distribution member would comprise silver. (The current distribution member need not be pure silver. An exterior layer of silver is all that is needed.) The silver screen is optionally included only to increase the surface area of the current distribution member.

Thus, in operation, an external source of electrical energy (not shown) may be connected to exterior connector 20 which is, in turn, electrically connected to the silver current distribution member 22, 23.

It should be noted with reference to the cathodic description of the sacrificial electrode described above that

the silver current distribution member shown could be a silver/silver chloride cathode. Electrode assembly 10, as depicted, would be placed in contact with a patient's skin and pressed with sufficient firmness so that pressure sensitive adhesive 16 would hold the drug reservoir 18 in contact therewith by means of flexible housing 12. Silver tab 20 would be electrically connected to a source of electrical energy, preferably a small battery. Utilization of a battery permits iontophoretic drug delivery without substantial interference with the normal activities of the patient.

It is within the contemplation of the present invention that stationary electrical supply sources be employed in which case it would likely be necessary for the patient to be substantially immobilised. Although not depicted, a second indifferent or cooperating electrode would then be placed in contact with the patient's skin at a site separate from but electrically adjacent to the site on which electrode assembly 10 has been placed. Upon connection to a source of electrical energy, migration of charged species from reservoir 18 would occur.

Heretofore, the discussion has focussed upon the use of iontophoresis to treat humans. Obviously, the invention herein disclosed could be used with animals and should not be limited to humans.

The present invention will now be illustrated by the following example I which should not be employed so as to limit the scope of this invention:

EXAMPLE I

A number of anode/cathode drug combinations and drug concentrations were evaluated with respect to their minimization of the production of undesired species. In particular, drug reservoir pH changes were measured, from which hydronium ion delivery rates were computed. (The drug passed through a PVA membrane and was delivered onto 0.06 M Na Cl). Further, using the mathematical treatment described above, drug delivery efficiencies were determined. Experimental conditions and efficiencies were measured for delivery of lithium ion, potassium ion and salicylate ion as indicated in Tables 1, 2 and 3.

A number of observations may be made about the systems tested. Systems numbered 1-6 of Table 1 are basically prior art systems.

Undesirable water electrolysis products are produced and thus pH is shown to change fairly substantially as in "pH" column for systems 1 and 2 of Table 2 and 1 of Table 3.

As evidenced in Tables 2 and 3, hydronium delivery rates were generally higher, and drug delivery efficiencies were generally lower for prior art systems versus systems employing the present invention. For example, systems 1 and 2 in Table 2 should be compared with 3-6 in Table 2 and system 1 in Table 3 with 2-8 in Table 3.

## Table 1

### Summary of Experimental Conditions

| Drug | Reservoir Concentration (M) | Electrode Material | Electrode Reaction | Neutralization Reaction |
|---|---|---|---|---|
| 1. LiCl | 0.06 | Platinum | $H_2O \rightarrow 2H^+ + 1/2O_2 + 2e^-$ | None |
| 2. $LiNO_3$ | 0.06 | Platinum | " | " |
| 3. LiCl | 0.06 | S. Steel | " | " |
| 4. KCl | 0.06 | Platinum | " | " |
| 5. $K_3Fe(CN)_6$ | 0.02 | Platinum | " | " |
| 6. NaSal | 0.06 | Platinum | $H_2O + e^- \rightarrow OH^- + 1/2\ H_2$ | " |
| 7. $LiNO_3$ | 0.06 | Silver | $Ag \rightarrow Ag^+ + e^-$ | None |
| 8. $LiNO_3$ | 0.06 | Copper | $Cu \rightarrow Cu^+ + e^-$ | " |
| 9. $KNO_3$ | 0.06 | Silver | $Ag \rightarrow Ag^+ + e-$ | " |
| 10. NaSal | 0.06 | Ag/AgCl | $AgCl + e^- \rightarrow Ag + Cl^-$ | " |
| 11. HSal | Solid | Ag/AgCl | $AgCl + e^- \rightarrow Ag + Cl$ | " |
| 12. $Cu(Sal)_2$ | 0.05 | Silver | $Cu^{2+} + 2e^- \rightarrow Cu$ | " |
| 13. AgSal | 0.04 | Silver | $Ag^+ + e^- \rightarrow Ag$ | " |
| 14. AgSal | Solid | Platinum | $Ag^+ + e^- \rightarrow Ag$ | " |

EP 0 182 520 B2

Table 1 (cont'd)

Summary of Experimental Conditions

| Drug | Reservoir Concentration (M) | Electrode Material | Electrode Reaction | Neutralization Reaction |
|------|------|------|------|------|
| 15. LiCl | 0.06 | Silver | $Ag \rightarrow Ag^+ + e^-$ | $Ag^+ + Cl^- \rightarrow AgCl$ |
| 16. $Li_2CO_3$ | 0.03 | Silver | $Ag \rightarrow Ag^+ + e-$ | $2Ag^+ + CO_3^{2-} \rightarrow Ag_2CO_3$ |
| 17. LiCl | 0.06 | Copper | $Cu \rightarrow Cu^+ + e^-$ | $Cu^+ + Cl^- \rightarrow CuCl$ |
| 18. LiOH | 0.06 | Platinum | $H_2O \rightarrow 2H^+ + 1/2\ O_2 + 2e^-$ | $H^+ + OH^- \rightarrow H_2O$ |
| 19. KCl | 0.06 | Silver | $Ag \rightarrow Ag^+ + e^-$ | $Ag^+ + Cl^- \rightarrow AgCl$ |
| 20. $K_3Fe(CN)_6$ | 0.02 | Silver | $Ag \rightarrow Ag^+ + e^-$ | $3Ag^+ + Fe(CN)_6^{3-} \rightarrow Ag_3Fe(CN)_6$ |
| 21. $K_3Fe(CN)_6$ | 0.02 | Copper | $Cu \rightarrow Cu^+ + e^-$ | $3Cu^+ + Fe(CN)_6^{3-} \rightarrow Ag_3Fe(CN)_6$ |
| 22. AgSal | Solid | Ag/AgCl | $AgCl + e^- \rightarrow Ag + Cl^-$ | $Cl^- + AgSal \rightarrow AgCl + Sal^-$ |
| 23. HSal | Solid | Platinum | $H_2O + e^- \rightarrow OH^- + 1/2\ H_2$ | $OH^- + HSal \rightarrow H_2O + Sal^-$ |

NB. Sal = salicylate, S.steel = stainless steel

Systems 20 and 22 are in accordance with the invention

Table 2

| Comparison of Potassium Drug Delivery Systems* | | | | | |
|---|---|---|---|---|---|
| Drug | Anode Material | %Drug Delivery Efficiency | Hydronium Delivery Rate(moles/hr) | pH | |
| | | | | Initial Value | After 6 hrs |
| 1. KCl | Platinum | 33.7 | $1.4 \times 10^{-6}$ | 5.8 | 2.7 |
| 2. $K_3Fe(CN)_6$ | Platinum | 28.6 | $2.1 \times 10^{-6}$ | 6.4 | 2.6 |
| 3. $KNO_3$ | Silver | 36.2 | $5.4 \times 10^{-9}$ | 5.8 | 4.3 |
| 4. KCl | Silver | 39.1 | $<10^{-9}$ | 6.0 | 5.6 |
| 5. $K_3Fe(CN)_6$ | Silver | 34.3 | $1.0 \times 10^{-8}$ | 6.4 | 5.9 |
| 6. $K_3Fe(CN)_6$ | Copper | 33.8 | $8.9 \times 10^{-10}$ | 7.2 | 4.9 |

\* All results obtained at a current of 1 mA.
System 5 is in accordance with the invention

Table 3

| Comparison of Salicylate Drug Delivery Systems* | | | | | |
|---|---|---|---|---|---|
| Drug | Cathode Material | %Drug Delivery Efficiency | Hydronium Delivery Rate(moles/hr) | pH | |
| | | | | Initial Value | After 6 hrs |
| 1. NaSal | Platinum | 25.9 | $4.6 \times 10^{-6}$ | 6.0 | 11.5 |
| 2. NaSal | Ag/AgCl | 27.8 | $<10^{-9}$ | 6.5 | 5.6 |
| 3. HSal | Ag/AgCl | 20.9 | $<10^{-9}$ | 3.5 | 2.9 |
| 4. $Cu(Sal)_2$ | Silver | 28.3 | $< 10^{-9}$ | 4.5 | 4.5 |
| 5. AgSal | Silver | 28.2 | $1.0 \times 10^{-7}$ | 8.6 | 8.7 |
| 6. AgSal | Platinum | 24.9 | $3.3 \times 10^{-8}$ | 5.2 | 8.1 |
| 7. AgSal | Ag/AgCl | 25.0 | $2.9 \times 10^{-10}$ | 5.2 | 5.4 |
| 8. HSal | Platinum | 28.1 | $8.6 \times 10^{-11}$ | 2.8 | 3.4 |

\* All results obtained at a current of 1 mA.
System 7 is in accordance with the invention

**Claims**

1. An iontophoretic drug delivery apparatus comprising an iontophoretic drug delivery electrode assembly (10) itself comprising an electrode member (22,23) in electrical connection to a source (18,19,21) for an ionic drug species (19) to be delivered iontophoretically and for counterions therefor, said assembly containing at least one electrochemically active component which is oxidised or reduced during iontophoretic drug delivery or which oxidises or reduces other available species, in electrical connection to said source, and at least one of said electrochemically active component and said counterions being such that in the operation of said assembly generation of ionic species at said electrode member is substantially avoided or ionic species generated at said electrode member are substantially entirely converted by species present or generated in or at said source and/or said electrochemically active component into species having lower susceptibility to iontophoretic delivery from said assembly,
**characterised in that**
said assembly comprises as said electrochemically active component said electrode member (22,23) which is a cathode which is silver chloride in contact with silver,
said counterions for said ionic drug species (19) present in said source comprise silver ions,
and said source comprises a gel reservoir containing therein the ionic drug species (19) to be delivered iontophoretically,

whereby during operation of said assembly water electrolysis at said electrode member is substantially avoided.

2. An apparatus as claimed in claim 1 further comprising support means (12) for supporting said source and/or said electrode member.

3. An apparatus as claimed in either of claims 1 and 2, wherein said drug is in said gel at a concentration of less than 0.5 molar.

4. An iontophoretic drug delivery electrode assembly (10) comprising an electrode member (22,23) in electrical connection to a source (18,19,21) for an ionic drug species (19) to be delivered iontophoretically and for counterions therefor, said assembly containing at least one electrochemically active component which is oxidised or reduced during iontophoretic drug delivery or which oxidises or reduces other available species, in electrical connection to said source, and at least one of said electrochemically active component and said counterions being such that in the operation of said assembly generation of ionic species at said electrode member is substantially avoided or ionic species generated at said electrode member are substantially entirely converted by species present or generated in or at said source and/or said electrochemically active component into species having lower susceptibility to iontophoretic delivery from said assembly,
**characterised in that**
said assembly comprises as said electrochemically active component said electrode member (22,23) which is an intercalation compound or amalgam,
whereby during operation of said assembly water electrolysis at said electrode member is substantially avoided.

5. An iontophoretic drug delivery apparatus comprising an electrode assembly (10) as claimed in claim 4.

6. An apparatus as claimed in claim 5, further comprising support means (12) for supporting said source and/or said electrode member.

7. An apparatus as claimed in either of claims 5 and 6, wherein said source comprises a gel reservoir containing therein the ionic drug to be delivered iontophoretically.

8. An apparatus as claimed in claim 7, wherein said drug is in said gel at a concentration of less than 0.5 molar.

9. An apparatus as claimed in either one of claims 7 and 8, wherein said counterions comprise alkali metal ions.

10. An apparatus as claimed in any one of claims 7 to 9, wherein said intercalation compound is an intercalate of a material selected from sodium tungstate, sodium vanadate ($NaV_6O_3$) and graphite.

11. An apparatus as claimed in any one of claims 7 to 9 wherein said amalgam is a mercury/cadmium amalgam.

12. An iontophoretic drug delivery electrode assembly (10) comprising an electrode member (22,23) in electrical connection to a source (18,19,21) for an ionic drug species (19) to be delivered iontophoretically and for counterions therefor, said assembly containing at least one electrochemically active component which is oxidised or reduced during iontophoretic drug delivery or which oxidises or reduces other available species, in electrical connection to said source, and at least one of said electrochemically active component and said counterions being such that in the operation of said assembly generation of ionic species at said electrode member is substantially avoided or ionic species generated at said electrode member are substantially entirely converted by species present or generated in or at said source and/or said electrochemically active component into species having lower susceptibility to iontophoretic delivery from said assembly,
**characterised in that**
said assembly comprises as said electrochemically active component said electrode member (22,23) which is a silver, tin, zinc, nickel or manganese anode,
and said drug counterions comprise ferrocyanide or ferricyanide ions,
whereby during operation of said assembly water electrolysis at said electrode member is substantially avoided.

13. An iontophoretic drug delivery apparatus comprising an electrode assembly (10) as claimed in claim 12.

14. An apparatus as claimed in claim 13 further comprising support means (12) for supporting said source and/or said electrode member.

**15.** An apparatus as claimed in either of claims 13 and 14, wherein said source (18) comprises a gel reservoir containing therein the drug to be delivered iontophoretically.

**16.** An apparatus as claimed in claim 15, wherein said drug is in said gel at a concentration of less than 0.5 molar.

**Patentansprüche**

**1.** Vorrichtung zur iontophoretischen Verabreichung von Medikamenten mit einer Elektrodenanordnung (10) zur iontophoretischen Verabreichung von Medikamenten, die selbst ein Elektrodenelement (22, 23) in elektrisch leitender Verbindung mit einer Source (18, 19, 21) für ein iontophoretisch zu verabreichendes, ionisches Medikament und für die Gegenionen davon umfasst,
wobei die Anordnung wenigstens eine elektrochemisch aktive Komponente, die während der iontophoretischen Verabreichung des Medikaments oxidiert oder reduziert wird,
oder die andere verfügbare Spezies oxidiert oder reduziert, in elektrisch leitender Verbindung mit der Source enthält, und
wobei wenigstens eine der elektrochemisch aktiven Komponenten und die Gegenionen so gewählt sind, dass bei Betätigung der Anordnung die Erzeugung ionischer Spezies an dem Elektrodenelement im wesentlichen vermieden wird oder die an dem Elektrodenelement erzeugten ionischen Spezies im wesentlichen vollständig durch in oder bei der Source vorliegende oder gebildete Spezies und/oder durch die elektrochemisch aktive Komponente in Spezies umgewandelt werden, die eine niedrigere Suszeptibilität für eine iontophoretische Abgabe durch die Vorrichtung aufweisen, **dadurch gekennzeichnet,**
**dass** die Anordnung als die elektrochemisch aktive Komponente das Elektrodenelement (22, 23) enthält, das eine Kathode ist, die aus Silberchlorid in Kontakt mit Silber ist,
die Gegenionen für die ionische Medikamentenspezies (19), welche in der Source vorliegen, Silberionen enthalten, und die Source ein Gelreservoir umfasst, in der das iontophoretisch abzuscheidende Medikament enthalten ist,
wobei bei Betätigung der Anordnung eine Wasserelektrolyse an dem Elektrodenelement im wesentlichen vermieden wird.

**2.** Vorrichtung nach Anspruch 1, die weiterhin Haltemittel (12) zum Halten der Source und/oder des Elektrodenelements umfasst.

**3.** Vorrichtung nach Anspruch 1 oder 2, worin das Medikament in dem Gel in einer Konzentration von weniger als 0,5 molar enthalten ist.

**4.** Elektrodenanordnung (10) zur iontophoretischen Verabreichung von Medikamenten, umfassend eine Elektrodenelement (22, 23) in elektrisch leitender Verbindung mit einer Source (18, 19, 21) für ein iontophoretisch zu verabreichendes, ionisches Medikament und für die Gegenionen davon,
wobei die Anordnung wenigstens eine elektrochemisch aktive Komponente, die während der iontophoretischen Verabreichung des Medikaments oxidiert oder reduziert wird,
oder die andere verfügbare Spezies oxidiert oder reduziert, in elektrisch leitender Verbindung mit der Source enthält, und
wobei wenigstens eine der elektrochemisch aktiven Komponenten und die Gegenionen so gewählt sind, dass bei Betätigung der Anordnung die Erzeugung ionischer Spezies an dem Elektrodenelement im wesentlichen vermieden wird oder die an dem Elektrodenelement erzeugten ionischen Spezies im wesentlichen vollständig durch in oder bei der Source vorliegende oder gebildete Spezies und/oder durch die elektrochemisch aktive Komponente in Spezies umgewandelt werden, die eine niedrigere Suszeptibilität für eine iontophoretische Abgabe durch die Vorrichtung aufweisen, **dadurch gekennzeichnet, dass**
die Anordnung als die elektrochemisch aktive Komponente das Elektrodenelement (22, 23) enthält, das aus einer Einlagerungsverbindung oder Amalgam ist,
wobei bei Betätigung der Anordnung eine Wasserelektrolyse an dem Elektrodenelement im wesentlichen vermieden wird.

**5.** Vorrichtung zur iontophoretischen Verabreichung von Medikamenten mit einer Elektrodenanordnung (10) nach Anspruch 4.

**6.** Vorrichtung nach Anspruch 5, welche weiterhin Haltemittel (12) zum Halten der Source und/oder des Elektrodenelements umfasst.

**7.** Vorrichtung nach Anspruch 5 oder 6, worin die Source ein Gelreservoir umfasst, in der das iontophoretisch abzuscheidende, ionische Medikament enthalten ist.

**8.** Vorrichtung nach Anspruch 7, worin das Medikament in dem Gel in einer Konzentration von weniger als 0,5 molar enthalten ist.

**9.** Vorrichtung nach Anspruch 7 oder 8, worin die Gegenionen Alkalimetallionen enthalten.

**10.** Vorrichtung nach einem der Ansprüche 7 bis 9, worin die Einlagerungsverbindung eine Einlagerung eines Materials, ausgewählt unter Natriumwolframat, Natriumvanadat ($NaV_6O_3$) und Graphit, ist.

**11.** Vorrichtung nach einem der Ansprüche 7 bis 9, worin das Amalgam Quecksilber/Cadmiumamalgam ist.

**12.** Elektrodenanordnung (10) zur iontophoretischen Verabreichung von Medikamenten, umfassend
ein Elektrodenelement (22, 23) in elektrisch leitender Verbindung mit einer Source (18, 19, 21) für ein ionthophoretisch zu verabreichendes, ionisches Medikament und für die Gegenionen davon,
wobei die Anordnung wenigstens eine elektrochemisch aktive Komponente, die während der iontophoretischen Verabreichung des Medikaments oxidiert oder reduziert wird,
oder die andere verfügbare Spezies oxidiert oder reduziert, in elektrisch leitender Verbindung mit der Source enthält, und
wobei wenigstens eine der elektrochemisch aktiven Komponenten und die Gegenionen so gewählt sind, dass bei Betätigung der Anordnung die Erzeugung ionischer Spezies an dem Elektrodenelement im wesentlichen vermieden wird oder die an dem Elektrodenelement erzeugten ionischen Spezies im wesentlichen vollständig durch in oder bei der Source vorliegende oder gebildete Spezies und/oder durch die elektrochemisch aktive Komponente in Spezies umgewandelt werden, die eine niedrigere Suszeptibilität für eine iontophoretische Abgabe durch die Vorrichtung aufweisen, **dadurch gekennzeichnet,**
**dass** die Anordnung als die elektrochemisch aktive Komponente das Elektrodenelement (22, 23) enthält, das eine Silber-, Zinn-, Nickel- oder Mangan-Anode ist,
und die Medikamentengegenionen Ferrocyanid- oder Ferricyanidionen enthalten,
wobei bei Betätigung der Anordnung eine Wasserelektrolyse an dem Elektrodenelement im wesentlichen vermieden wird.

**13.** Vorrichtung zur iontophoretischen Verabreichung von Medikamenten, umfassend eine Elektrodenanordnung (10) nach Anspruch 12.

**14.** Vorrichtung nach Anspruch 13, welche weiterhin Haltemittel (12) zum Halten der Source und/oder des Elektrodenelements umfasst.

**15.** Vorrichtung nach Anspruch 13 oder 14, worin die Source ein Gelreservoir umfasst, in der das iontophoretisch abzuscheidende, ionische Medikament enthalten ist.

**16.** Vorrichtung nach Anspruch 15, worin das Medikament in dem Gel in einer Konzentration von weniger als 0,5 molar enthalten ist.

## Revendications

**1.** Dispositif pour administration iontophorétique de médicaments comprenant un assemblage d'électrode (10) pour administration iontophorétique de médicaments comprenant lui-même un élément d'électrode (22, 23) relié électriquement à une source (18, 19, 21) d'une espèce médicamenteuse ionique (19) à administrer par voie iontophorétique et des contre-ions correspondants, ledit assemblage contenant au moins un composant actif électrochimiquement qui est oxydé ou réduit pendant l'administration iontophorétique du médicament ou qui oxyde ou réduit d'autres espèces présentes, connecté électriquement à ladite source, et au moins l'un desdits composants actifs electrochimiquement et des dits contre-ions étant tel que lorsque ledit assemblage est en fonctionnement, la génération des espèces ioniques au niveau dudit élément d'électrode est pratiquement inhibée ou les espèces ioniques générées au niveau dudit élément d'électrode sont pratiquement entièrement converties par les espèces présentes ou générées dans ou au niveau de ladite source et/ou ledit composant électrochimiquement actif en espèces ayant une moindre susceptibilité d'être administrées par voie iontophorétique à partir dudit assemblage,

**caractérisé en ce que**

ledit assemblage comprend en tant que composant électrochimiquement actif ledit élément d'électrode (22, 23) qui est une cathode constituée de chlorure d'argent en contact avec de l'argent,

lesdits contre-ions pour lesdites espèces médicamenteuses ioniques (19) présents dans ladite source comprennent des ions argent,

et ladite source comprend un réservoir à gel contenant les espèces médicamenteuses ioniques (19) à administrer par voie iontophorétique,

et où pendant le fonctionnement dudit assemblage l'électrolyse de l'eau au niveau dudit élément d'électrode est pratiquement inhibée.

2. Dispositif selon la revendication 1, comprenant également des moyens de support (12) pour maintenir ladite source et/ou ledit élément d'électrode.

3. Dispositif selon la revendication 1 ou 2, selon lequel ledit médicament est présent dans ledit gel à une concentration inférieure à 0,5 molaire.

4. Assemblage d'électrode (10) pour administration d'un médicament par voie iontophorétique comprenant un élément d'électrode (22, 23) relié électriquement à une source (18, 19, 21) d'une espèce médicamenteuse ionique (19) à administrer par voie iontophorétique et de ses contre-ions, ledit assemblage contenant au moins un composant électrochimiquement actif qui est oxydé ou réduit pendant l'administration iontophorétique du médicament ou qui oxyde ou réduit d'autres espèces présentes, relié électriquement à ladite source, et au moins un dudit composant électrochimiquement actif et desdits contre-ions étant tel que lors du fonctionnement dudit assemblage la production d'espèces ioniques au niveau dudit élément d'électrode est pratiquement inhibée ou les espèces ioniques générées au niveau dudit élément d'électrode sont pratiquement entièrement converties par les espèces présentes ou générées dans ou au niveau de ladite source et/ou ledit composant électrochimiquement actif en espèces ayant une moindre susceptibilité d'être administrées par voie iontophorétique à partir dudit assemblage,

**caractérisé en ce que**

ledit assemblage comprend en tant que composant électrochimiquement actif ledit élément d'électrode (22, 23) qui est un composé d'intercalation ou un amalgame,

et dans lequel lors du fonctionnement dudit assemblage l'électrolyse de l'eau au niveau dudit élément d'électrode est pratiquement inhibée.

5. Dispositif d'administration de médicaments par voie iontophorétique comprenant un assemblage d'électrode (10) selon la revendication 4.

6. Dispositif selon la revendication 5, comprenant également des moyens de support (12) pour maintenir ladite source et/ou ledit élément d'électrode.

7. Dispositif selon l'une des revendications 5 ou 6, selon lequel ladite source comprend un réservoir de gel renfermant le médicament ionique à administrer par voie iontophorétique.

8. Dispositif selon la revendication 7, selon lequel ledit médicament se trouve dans ledit gel à un concentration de moins de 0,5 molaire.

9. Dispositif selon l'une quelconque des revendications 7 et 8, selon lequel lesdits contre-ions comprennent des ions de métal alcalin.

10. Dispositif selon l'une quelconque des revendications 7 à 9, selon lequel ledit composé d'intercalation est un intercalate d'un matériau choisi parmi le tungstate de sodium, le vanadate de sodium ($NaV_6O_3$) et le graphite.

11. Dispositif selon l'une quelconque des revendications 7 à 9 selon lequel ledit amalgame est un amalgame mercure/cadmium.

12. Assemblage d'électrode (10) pour l'administration d'un médicament par voie iontophorétique comprenant un élément d'électrode (22, 23) connecté électriquement à une source (18, 19, 21) d'une espèce médicamenteuse ionique (19) à administrer par voie iontophorétique et de ses contre-ions, ledit assemblage contenant au moins un composant électrochimiquement actif qui est oxydé ou réduit pendant l'administration du médicament par voie iontophorétique ou qui oxyde et réduit d'autres espèces présentes, connecté électriquement à ladite source, et au moins l'un dudit composant actif électrochimiquement et desdits contre-ions étant tel que lors du fonctionnement dudit assemblage la génération d'espèces ioniques au niveau dudit élément d'électrode est pratiquement inhibée ou les espèces ioniques générées au niveau dudit élément d'électrode sont pratiquement entièrement converties par les espèces présentes ou générées dans ou au niveau de ladite source et/ou ledit composant électrochimiquement actif en espèces ayant une moindre susceptibilité à être administrées par voie iontophorétique à partir dudit assemblage,

**caractérisé en ce que**

ledit assemblage comprend en tant que composant électrochimiquement actif ledit élément d'électrode (22, 23) qui est une anode en argent, étain, zinc, nickel ou manganèse,

et **en ce que** lesdits contre-ions du médicament comprennent des ions ferrocyanure ou ferricyanure,

et dans lequel pendant le fonctionnement dudit assemblage l'électrolyse de l'eau au niveau dudit élément d'électrode est pratiquement inhibée.

13. Dispositif d'administration de médicament par voie iontophorétique comprenant un assemblage d'électrode (10) selon la revendication 12.

14. Dispositif selon la revendication 13 comprenant également des moyens de support (12) pour maintenir ladite source et/ou ledit élément d'électrode.

15. Dispositif selon l'une quelconque des revendications 13 et 14, selon lequel ladite source (18) comprend un réservoir à gel renfermant le médicament à être administré par voie iontophorétique.

16. Dispositif selon la revendication 15, selon lequel ledit médicament se trouve dans ledit gel à une concentration de moins de 0,5 molaire.

FIG.1.